# EUROPÄISCHE PATENTSCHRIFT

(11) **EP 1 764 048 B1**
(45) Veröffentlichungstag und Bekanntmachung des Hinweises auf die Patenterteilung: **10.03.2010**
(21) Anmeldenummer: 06016992.7
(22) Anmeldetag: 16.08.2006
(51) Int. Cl.: A61B 17/70, A61B 17/80

(54) **Ventrale Platte**
Ventral plate
Plaque ventrale

(30) Priorität: 16.09.2005 DE 102005044532
(43) Veröffentlichungstag der Anmeldung: 21.03.2007
(73) Patentinhaber: Ulrich GmbH & Co. KG, 89081 Ulm (DE)
(72) Erfinder: Khodadadyan-Klostermann, Cyrus Dr., 32756 Detmold (DE); Neumann, Carsten Dr. med., 93007 Bad Abbach (DE)
(74) Vertreter: Hentrich, Swen

(56) Entgegenhaltungen:
- DE-T2- 69 918 998
- US-A1- 2005 177 161

## Beschreibung

Die Erfindung betrifft eine ventrale Platte zur Stabilisierung der Wirbelsäule, mit einem eine Mehrzahl von Öffnungen aufweisenden Plattenkörper zur Aufnahme von die Öffnungen durchsetzenden, in die Wirbelkörper benachbarter Wirbel einzuschraubender Knochenschrauben.

Knochenplatten zur Osteosynthese sind seit langem bekannt und werden insbesondere eingesetzt, um Bruchstellen bei Knochenbrüchen zu überdecken. Darüber hinaus ist ein Einsatz auch in der Wirbelsäulenchirurgie erfolgt zur Versteifung benachbarter Wirbelkörper. Allerdings ist es nachteilig, dass bei bekannten Knochenplatten die eingangs erwähnte Überdeckung erfolgt, also der Operateur keinen Einblick in die von der Platte überdeckten Bereiche hat, was insbesondere in der Wirbelsäulenchirurgie nachteilig ist, wenn nachfolgend nach der Platzierung der Platte die Operation fortgesetzt werden muss zum Einbringen von Knochen, Knochenzement oder eines anderen Wirbelsäulenersatzelementes. Die Patentschrift DE 699 18 998 T2 offenbart eine Wirbelsäulenosteosynthesevorrichtung zur vorderen Fixierung mittels einer Platte. Diese Vorrichtung umfasst einen ersten Arm, in den Knochenschrauben eingesetzt werden können, und eine längsförmige Platte, die einteilig mit dem ersten Arm ausgebildet ist, sowie einen zweiten Arm, in der Knochenschrauben eingesetzt werden können, wobei die längsförmige Platte die Arme steif verbinden kann. Weiterhin weist diese Vorrichtung Mittel für die Regulierung der Längsrichtung des zweiten Arms auf der Platte auf. Die Offenlegungsschrift US 2005/0177161 A1 offenbart eine ventrale Platte zur Stabilisierung der Wirbelsäule mit ineinander greifenden Komponenten, um die Zuverlässigkeit der Stabilisierung zu erhöhen, wobei die Platte C-förmig ausgestaltet ist.

Der Erfindung liegt daher die Aufgabe zugrunde, eine ventrale Platte der eingangs Art so auszubilden, dass diese die Durchführung der Operation für den Operateur erleichtert.

Diese Aufgabe wird bei einer Platte der eingangs genannten Art dadurch gelöst, dass der Plattenkörper U-förmig gestaltet ist mit einem Basissteg und zwei davon abstehenden Schenkeln, in denen die Öffnungen ausgebildet sind, und dass eine Auxiliarplatte zur Befestigung an den Schenkeln vorgesehen ist zur Überbrückung von deren gegenseitigem Abstand.

Diese Auxiliarplatte stellt sicher, dass der zwischen den Schenkeln gegebene Abstand die Zugänglichkeit in den Raum hinter der Platte anfänglich gegeben ist, wobei durch die Auxiliarplatte, die zu einem späteren Zeitpunkt an der Platte befestigt werden kann, deren mechanische Eigenschaften verändert werden, insbesondere die Steifigkeit und Belastbarkeit erhöht wird, wenn die Zugänglichkeit nicht mehr erforderlich ist.

Mit einer derartigen Gestaltung ist der Vorteil verbunden, dass zwar über die die Öffnungen durchsetzenden Knochenschrauben die Platte an benachbarten Wirbelkörpern fixiert werden kann, dass aber der Bereich zwischen den Wirbelkörpern unverändert gut einsehbar und auch zugänglich bleibt, weil der Bereich unmittelbar zwischen den Öffnungen ausgespart ist. Durch die Schenkel bleibt dabei eine grosszügig bemessene Anlagefläche der Platte an den Wirbelkörpern erhalten, so dass Kräfte ohne grosse Punktbelastungen übertragen werden können.

Um eine verbesserte Anlage der Platte bei ventraler Positionierung an den Wirbelkörpern zu erreichen, ist vorgesehen, dass die Oberfläche auf der den Wirbelkörpern zuweisenden Seiten im Querschnitt konkav gewölbt ist. Eine Erleichterung der korrekten Positionierung der Platte relativ zu den Wirbelkörpern und ggfs. darin bereits eingesetzter Knochenschrauben ist gegeben, wenn in dem Basissteg ein Langloch sowie ein Rundloch ausgebildet ist. Damit ist insbesondere die Möglichkeit geschaffen, eine Befestigung der ventralen Platte an den Wirbelkörper nicht nur durch die die Öffnungen durchsetzenden Knochenschrauben zu erzielen, sondern zusätzlich auch den Basissteg zu fixieren, wozu es dann vorteilhaft ist, wenn der Basissteg und die Schenkel mehrteilig gebildet sind, da so optional die Befestigung der Platte sukzessive an den Wirbelkörpern erfolgen kann, also beispielsweise zunächst der Basissteg oder die Schenkel an den Wirbelkörpern befestigt werden, um nachfolgend dann die Verbindung zu dem komplementären Bauteil herzustellen und dessen korrekte Ausrichtung zu bewirken.Natürlich bleibt die Möglichkeit erhalten, alternativ die vormatiert ausgelieferte Platte auch als Einheit zu befestigen. Zur sukzessiven Befestigung ist es günstig, wenn im Basissteg Rastsitze für die Schenkel ausgebildet sind, um so einen Zusammenhalt zwischen dem Basissteg und den Schenkeln sicher zu stellen, auch bevor die Knochenschrauben vollständig angezogen sind. Die durch das Langloch gewünschte und sicher gestellte Verschiebbarkeit bleibt auch bei der Verwendung und Ausbildung der Rastsitze erhalten, wenn im Langloch in dessen Längsrichtung Rastleisten ausgebildet sind zum Zusammenwirken mit an dem Schenkel ausgebildeten Rastzungen.

Vorteilhaft ist es weiterhin, wenn an jedem der Schenkel zwei der Öffnungen ausgebildet sind, da so die zweite Öffnung ermöglicht, dass die das Langloch oder das Rundloch durchsetzende Knochenschraube zugleich auch der Festlegung des Schenkels relativ zu dem Basissteg und relativ zu dem Wirbelkörper dienen kann.

Eine bessere, insbesondere formschlüssige Verbindung zwischen der Platte und der Auxiliarplatte wird erreicht, indem in den Schenkeln Aufnahmen ausgebildet sind zum Einsetzen der Auxiliarplatte.

Um für die Auxiliarplatte keine zusätzlichen Knochenschrauben vorsehen zu müssen, ist die Gestaltung so getroffen, dass in der Auxiliarplatte Durchgänge ausbildet sind, die bei in die Aufnahmen eingesetzter Auxiliarplatte mit den Öffnungen fluchten.

Besonders bevorzugt ist dabei, wenn die Öffnungen auf der den Wirbel zugewandten Seite konkav geformt sind, da dadurch die Möglichkeit besteht, die Orientierung der Knochenschrauben, insbesondere die Orientierung von deren Längsachse zu der Platte zu modifizieren und an die konkret vorliegenden anatomischen Gegebenheiten anzupassen.

Vorteilhaft ist es weiterhin, wenn die Schrauben in zwei Gestaltungen vorliegen, von denen eine einen konvex geformten Schraubenkopf und die andere einen Ringbund am Schraubenkopf aufweist.

Im folgenden wird die Erfindung an einem in der Zeichnungen dargestellten Ausführungsbeispiel näher erläutert; es zeigen:
- Fig. 1: eine perspektivische Ansicht einer erfindungsgemäßen ventralen Platte zusammen mit vier Knochenschrauben,
- Fig. 2: eine Explosionsdarstellung des Gegenstandes der Fig. 1,
- Fig. 3: eine Vorderansicht des Gegenstandes aus Fig. 1,
- Fig. 4: eine Seitenansicht des Gegenstandes aus Fig. 1,
- Fig. 5: eine Draufsicht des Gegenstandes aus Fig. 1,
- Fig. 6: den Schnitt VI-VI aus Fig. 5,
- Fig. 7: eine der Fig. 5 entsprechende Darstellung mit anderer Schnittlinie,
- Fig. 8: den Schnitt VIII-VIII aus Fig. 7,
- Fig. 9: eine schematische Darstellung der an dem Wirbelkörper befestigten ventralen Platte,
- Fig. 10: eine Sicht aus Richtung des Pfeiles X aus Fig. 9, und
- Fig. 11: eine der Fig. 10 entsprechende Darstellung mit der symbolisierten Kombination der Platte mit einem distrahierbaren Wirbelsäulenimplantat.

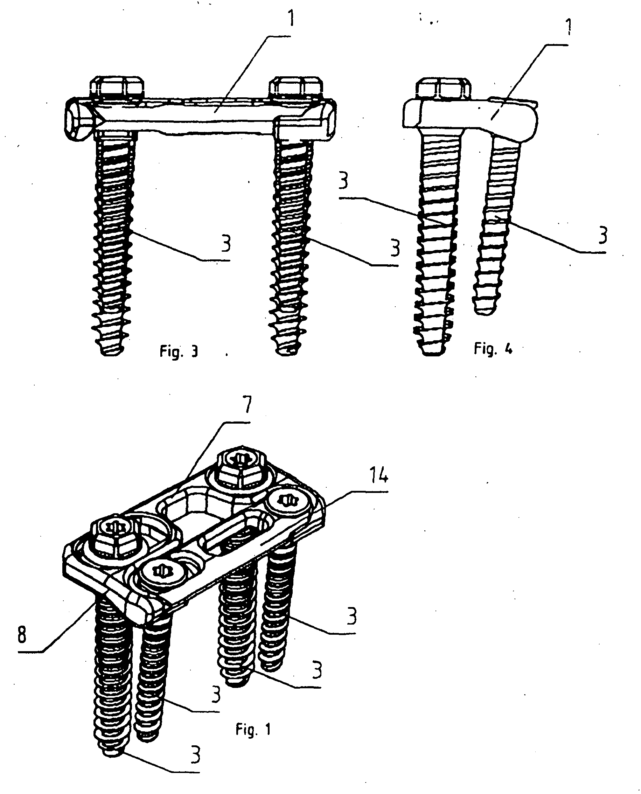

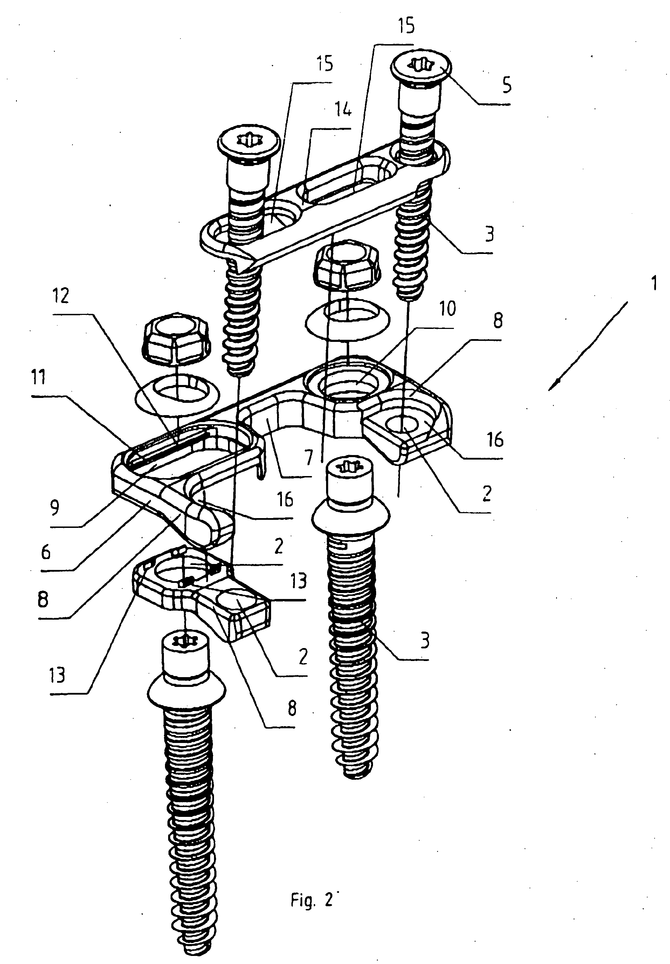

In der Zeichnung ist in den Fig. 1 bis 10 eine ventrale Platte 1 gezeigt, die der Stabilisierung der Wirbelsäule dient und dazu eine Mehrzahl von Öffnungen 2 in einem Plattenkörper aufweist, der von Knochenschrauben 3 durchsetzt wird, um die Platte 1 an den Wirbelkörpern 4 benachbarter Wirbel zu befestigen. Die Knochenschrauben 3 liegen dabei in zwei Gestaltungen vor, von denen eine einen konvex geformten Schraubenkopf und die andere einen Ringbund 5 am Schraubenkopf aufweist (Fig. 2).

Die Platte 1 besitzt einen U-förmig gestalteten Plattenkörper 6 mit einem Basissteg 7 und zwei davon abstehenden Schenkeln 8, in denen die Öffnungen 2 für die Knochenschrauben 3 ausgebildet sind, wobei die Oberfläche auf der den Wirbelkörper 4 zuweisenden Seite der Platte 1 im Querschnitt konkav gewölbt ist.

Insbesondere Fig. 2 läßt erkennen, dass in dem Basissteg 7 ein Langloch 9 sowie ein Rundloch 10 ausgebildet ist und der Basissteg 7 und die Schenkel 8 mehrteilig gebildet sind. Im Basissteg 7 sind Rastsitze 11 für die Schenkel 8 ausgebildet, wobei einer der Rastsitze 11 dem Langloch 9 zugeordnet ist, das dazu in dessen Längsrichtung Rastleisten 12 aufweist zum Zusammenwirken mit an dem Schenkel 8 ausgebildeten Rastzungen 13. In dem Schenkel 8 sind zwei der Öffnungen 2 ausgebildet.

Die Zeichnung läßt weiterhin erkennen, dass der Platte 1 eine Auxiliarplatte 14 zur Befestigung an den Schenkeln 8 zugeordnet ist, durch die der gegenseitige Abstand der Schenkel 8 überbrückt werden kann, was im Ergebnis einer Vergrößerung der Platte 1 mit der durch diese gebildeten Auflagefläche entspricht. In den Schenkeln 8 sind Aufnahmen 16 ausgebildet, in die die Auxiliarplatte 14 formschlüssig eingesetzt werden kann. Weiterhin sind in der Auxiliarplatte 14 Durchgänge 15 ausgebildet, die bei in die Aufnahme eingesetzter Auxiliarplatte mit den Öffnungen 2 fluchten, so dass im Ergebnis die eine in dem Schenkel 8 ausgebildete Öffnung von einer Knochenschraube 3 durchsetzt wird, die auch durch einen der Durchgänge 15 der Auxiliarplatte 14 greift, während die andere Öffnung 2 des Schenkels 8 von einer Knochenschraube 3 durchsetzt wird, die auch das Langloch 9 oder das Rundloch 10 des Basissteges 7 durchgreift.

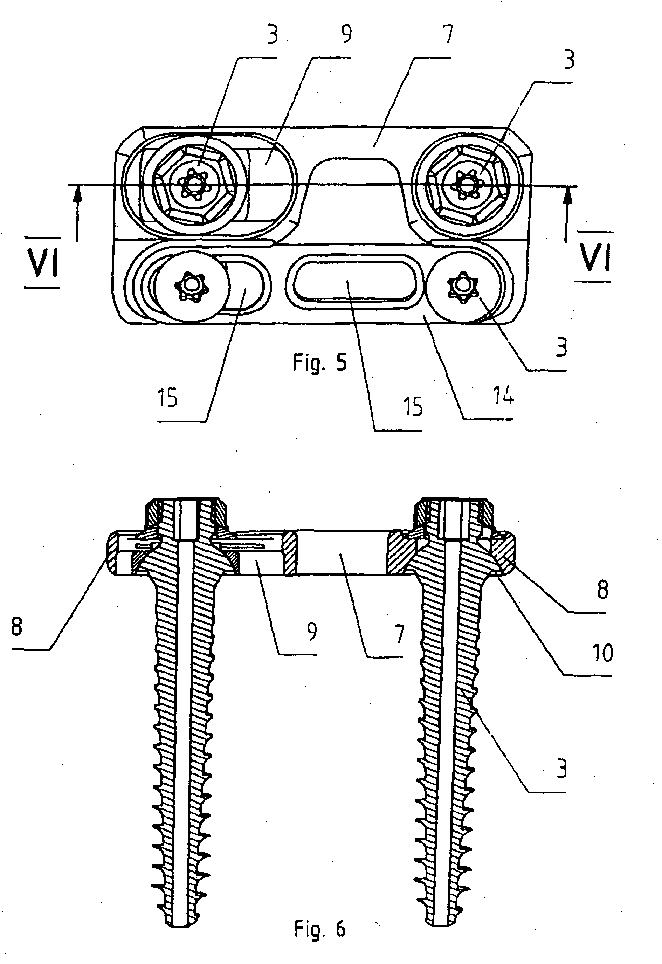

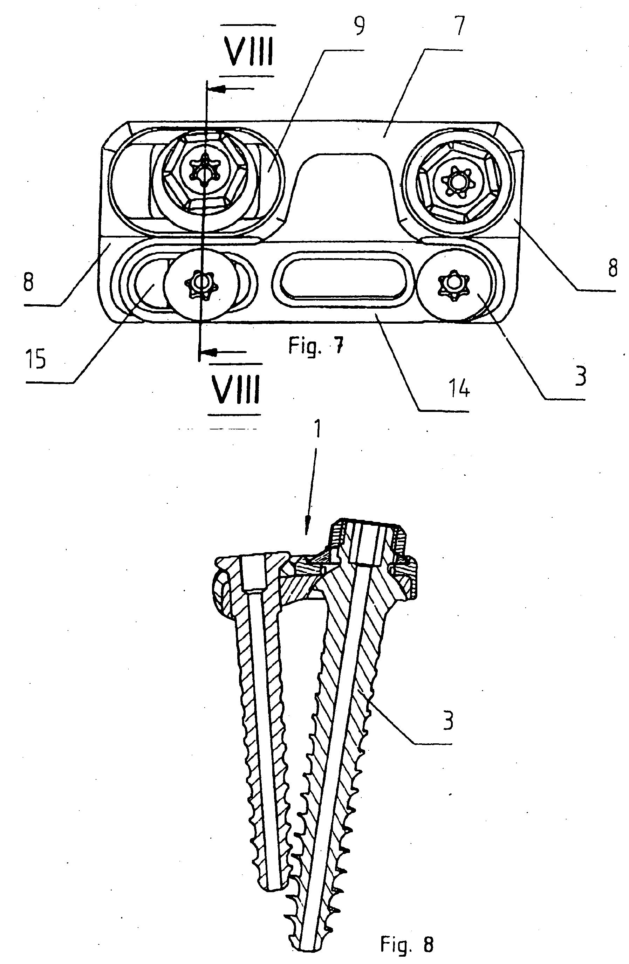

Die Fig. 6 und 8 lassen erkennen, dass die Öffnungen 2 auf der den Wirbel zugewandten Seite konkav geformt sind, so dass im Ergebnis die Knochenschrauben 3 mit dem konvex geformten Schraubenkopf in ihrer Orientierung innerhalb der Öffnungen 2 angepasst werden können.

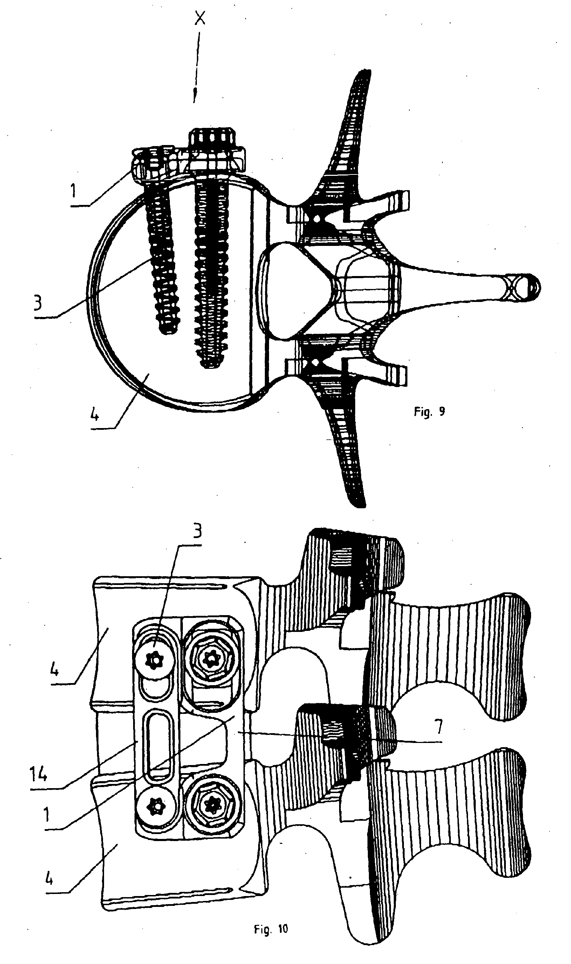

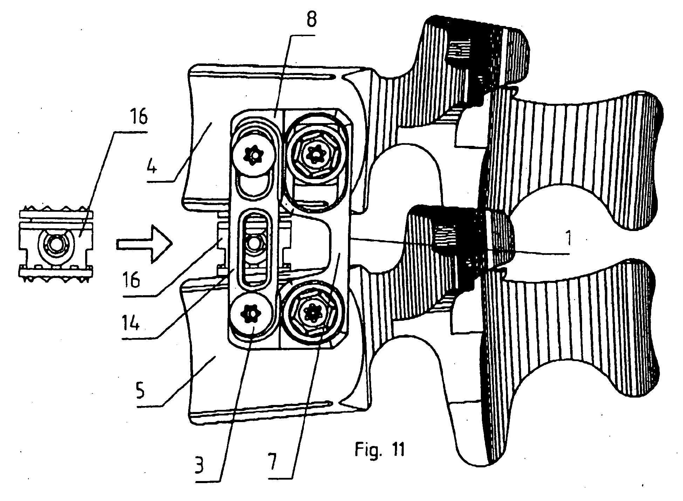

Die Fig. 9 bis 11 zeigen die erfindungsgemäße ventrale Platte 1 in ihrem Einsatzgebiet seitlich befestigt an zwei Wirbelkörpern 4 einer Wirbelsäule, wobei für den Operateur die Möglichkeit besteht, zunächst die Knochenschrauben 3 mit dem konvex geformten Schraubenkopf in die beiden benachbarten Wirbel einzuschrauben und sodann die Schenkel 8 und den Basissteg 7 aufzulegen mittels Muttern provisorisch oder endgültig zu sichern. Durch die C-förmige Gestaltung bleibt der Zugang zwischen den beiden Wirbelkörpern 4 und die Einsehbarkeit erhalten, so dass für den Operateur auch die Möglichkeit besteht, in einfacher Weise ein distrahierbares Element 16 als Bandscheibenersatz zwischen die Wirbelkörper 4 einzuführen. Bei entsprechender Längenausdehnung der Platte 1 zur Überbrückung der durch einen entfernten Wirbelkörper 4 gebildeten Lücke besteht auch die Möglichkeit einen Wirbelersatzkörper zwischen die entsprechend beabstandeten benachbarten Wirbelkörper 4 einzufügen. Zum Schluß wird die Auxiliarplatte 14 in die Aufnahmen 16 der Schenkel 8 eingesetzt und mittels der Knochenschraube 3 mit dem Ringbund 5 befestigt.

Fig. 6 verweist darauf, dass die Knochenschrauben 3 eine Kanülierung aufweisen, durch die zur Verbesserung des Sitzes der Knochenschrauben 3 im Wirbelkörper 4 ein Haftmittel, insbesondere Knochenzement verpresst werden kann. Der Austritt der Kanülierung muß dabei nicht axial erfolgen, sondern kann auch radial vorgesehen sein.

## Patentansprüche

1. Ventrale Platte zur Stabilisierung der Wirbelsäule, mit einem eine Mehrzahl von Öffnungen (2) aufweisenden Plattenkörper zur Aufnahme von die Öffnungen (2) durchsetzenden, in die Wirbelkörper (4) benachbarter Wirbel einzuschraubender Knochenschrauben (3), wobei der Plattenkörper U-förmig gestaltet ist mit einem Basissteg (7) und zwei davon abstehenden Schenkeln (8), in denen die Öffnungen (2) ausgebildet sind, **dadurch gekennzeichnet, dass** eine Auxiliarplatte (14) zur Befestigung an den Schenkeln (8) vorgesehen ist zur Überbrückung von deren gegenseitigem Abstand.

2. Platte nach Anspruch 1, **dadurch gekennzeichnet, dass** in den Schenkeln (8) Aufnahmen (16) ausgebildet sind zum Einsetzen der Auxiliarplatte (14).

3. Platte nach Anspruch 2, **dadurch gekennzeichnet, dass** in der Auxiliarplatte (14) Durchgänge (15) ausgebildet sind, die bei in die Aufnahmen (16) eingesetzter Auxiliarplatte (14) mit den Öffnungen (2) fluchten.

4. Platte nach einem der Ansprüche 1 bis 3, **dadurch gekennzeichnet, dass** die Öffnungen (2) auf der den Wirbeln zugewandten Seite konkav geformt sind.

5. Platte nach Anspruch 4, mit zugeordneten Schrauben, **dadurch gekennzeichnet, dass** die Schrauben (3) in zwei Gestaltungen vorliegen, von denen eine einen konvex geformten Schraubenkopf und die andere einen Ringbund am Schraubenkopf (5) aufweist.

6. Platte nach einem der Ansprüche 1 bis 5, **dadurch gekennzeichnet, dass** die Oberfläche auf der den Wirbelkörpern (4) zuweisenden Seite im Querschnitt konkav gewölbt ist.

7. Platte nach einem der Ansprüche 1 bis 6, **dadurch gekennzeichnet, dass** in dem Basissteg (7) ein Langloch (9) sowie ein Rundloch (10) ausgebildet ist.

8. Platte nach einem der Ansprüche 1 bis 7, **dadurch gekennzeichnet, dass** der Basissteg (7) und die Schenkel (8) mehrteilig gebildet sind.

9. Platte nach Anspruch 8, **dadurch gekennzeichnet, dass** im Basissteg (7) Rastsitze (11) für die Schenkel (8) ausgebildet sind.

10. Platte nach Anspruch 8, **dadurch gekennzeichnet, dass** einer der Rastsitze (11) dem Langloch (9) zugeordnet ist.

11. Platte nach Anspruch 10, **dadurch gekennzeichnet, dass** im Langloch (9) in dessen Längsrichtung Rastleisten (12) ausgebildet sind zum Zusammenwirken mit an dem Schenkel (8) ausgebildeten Rastzungen (13).

12. Platte nach einem der Ansprüche 8 bis 11, **dadurch gekennzeichnet, dass** in jedem der Schenkel (8) zwei der Öffnungen (2) ausgebildet sind.

## Claims

1. A ventral plate for stabilising the spinal column comprising a plate body having a plurality of openings (2) for receiving bone screws (3) which pass through the openings (2) and which are to be screwed into the vertebral bodies (4) of adjacent vertebrae, wherein the plate body is U-shaped with a base web (7) and two limbs (8) which project therefrom and in which the openings (2) are provided, **characterised in that** there is provided an auxiliary plate (14) for fixing to the limbs (8) for bridging over the mutual spacing thereof.

2. A plate according to claim 1 **characterised in that** provided in the limbs (8) are receiving means (16) for insertion of the auxiliary plate (14).

3. A plate according to claim 2 **characterised in that** provided in the auxiliary plate (14) are passages (15) which when the auxiliary plate (14) is inserted in the receiving means (16) are aligned with the openings (2).

4. A plate according to one of claims 1 to 3 **characterised in that** the openings (2) are concavely shaped on the side towards the vertebrae.

5. A plate according to claim 4 with associated screws **characterised in that** the screws (3) are present in two configurations of which one has a convexly shaped screw head and the other has an annular collar on the screw head (5).

6. A plate according to one of claims 1 to 5 **characterised in that** the surface on the side facing towards the vertebral bodies (4) are concavely curved in cross-section.

7. A plate according to one of claims 1 to 6 **characterised in that** a slot (9) and a round hole (10) are provided in the base web (7).

8. A plate according to one of claims 1 to 7 **characterised in that** the base web (7) and the limbs (8) are formed in a plurality of parts.

9. A plate according to claim 8 **characterised in that** latching seats (11) for the limbs (8) are provided in the base web (7).

10. A plate according to claim 8 **characterised in that** one of the latching seats (11) is associated with the slot (9).

11. A plate according to claim 10 **characterised in that** provided in the slot (9) in the longitudinal direction thereof are latching bars (12) for cooperation with latching tongues (13) provided on the limb (8).

12. A plate according to one of claims 8 to 11 **characterised in that** two of the openings (2) are provided in each of the limbs (8).

## Revendications

1. Plaque ventrale pour stabiliser la colonne vertébrale, qui comporte une pluralité de corps de plaque présentant des ouvertures (2) pour accueillir, la traversant, des vis à os (3) à visser dans les corps (4) des vertèbres proches, ces corps de plaque ayant une forme en U avec une barrette de base (7) et, partant de celle-ci en s'en éloignant, deux branches (8) dans lesquelles sont formées des ouvertures, cette plaque étant **caractérisée en ce qu'**il est prévu une plaque auxiliaire (14) à fixer sur les branches (8) pour ponter leur intervalle.

2. Plaque selon la revendication 1, **caractérisée en ce que** les branches (8) présentent des logements (16) pour accueillir la plaque auxiliaire.

3. Plaque selon la revendication 2, **caractérisée en ce** la plaque auxiliaire (14) présente des passages (15) qui sont à fleur avec les ouvertures (2) quand la plaque auxiliaire (14) se trouve dans les logements (16).

4. Plaque selon une des revendications 1 à 3, **caractérisée en ce que** les ouvertures (2) ont une forme concave du côté en regard des vertèbres.

5. Plaque selon la revendication 4, avec les vis associées, **caractérisée en ce que** ces vis (3) sont réalisées selon deux configurations, dont l'une présente une tête de vis de forme convexe tandis que l'autre présente un collet annulaire sur la tête de vis (5).

6. Plaque selon une des revendications 1 à 5, **caractérisée en ce que** la surface du côté en regard des corps de vertèbre (4) présente en section une forme concave.

7. Plaque selon une des revendications 1 à 6, **caractérisée en ce que** la barrette de base (7) présente un trou allongé (9) et un trou rond (10).

8. Plaque selon une des revendications 1 à 7, **caractérisée en ce que** la barrette de base (7) et les branches (8) sont en plusieurs parties.

9. Plaque selon la revendication 8, **caractérisée en ce que** la barrette de base (7) présente des sièges d'arrêt (11) pour les branches (8).

10. Plaque selon la revendication 8, **caractérisée en ce qu'**à un des sièges d'arrêt (11) est affecté le trou allongé (9).

11. Plaque selon la revendication 10, **caractérisée en ce que** le trou allongé (9) présente, selon sa direction longitudinale, des barrettes d'arrêt (12) prévues pour coopérer avec des languettes d'arrêt (13) situées sur les branches (8).

12. Plaque selon une des revendications 8 à 11, **caractérisée en ce que** dans chacune des branches (8) se trouvent deux ouvertures (2).
